# EUROPÄISCHE PATENTSCHRIFT

(11) **EP 0 898 613 B1**
(45) Veröffentlichungstag und Bekanntmachung des Hinweises auf die Patenterteilung: **26.07.2000**
(21) Anmeldenummer: 97920664.6
(22) Anmeldetag: 12.04.1997
(51) Int. Cl.: C11D 3/386, C11D 3/22

(54) **LÖSLICHKEITSVERBESSERTES ENZYMGRANULAT**
IMPROVED-SOLUBILITY ENZYME GRANULATE
GRANULE ENZYMATIQUE A SOLUBILITE AMELIOREE

(30) Priorität: 20.04.1996 DE 19615776
(43) Veröffentlichungstag der Anmeldung: 03.03.1999
(73) Patentinhaber: Henkel Kommanditgesellschaft auf Aktien, 40589 Düsseldorf-Holthausen (DE)
(72) Erfinder: PAATZ, Kathleen, D-40589 Düsseldorf (DE); RÄHSE, Wilfried, D-40589 Düsseldorf (DE); PICHLER, Werner, A-6250 Kundl (AT); UPADEK, Horst, D-40883 Ratingen (DE)
(86) Internationale Anmeldenummer: EP9701836
(87) Internationale Veröffentlichungsnummer: WO9740128

(56) Entgegenhaltungen:
- EP-A- 0 170 360
- DE-A- 4 041 752
- DE-A- 4 310 506
- DATABASE WPI Section Ch, Week 9020 Derwent Publications Ltd., London, GB; Class A97, AN 90-152265 XP002038187 & JP 02 097 388 A (SHOWA DENKO KK) , 9.April 1990
- DATABASE WPI Section Ch, Week 8246 Derwent Publications Ltd., London, GB; Class A97, AN 82-98956E XP002038188 & JP 57 165 495 A (SHOWA DENKO KK) , 12.Oktober 1982

## Beschreibung

Die vorliegende Erfindung betrifft ein Enzymgranulat, ein Verfahren zu seiner Herstellung und die Verwendung des Granulats in festen, insbesondere teilchenförmigen Wasch- und Reinigungsmitteln.

Enzyme, insbesondere Proteasen, finden ausgedehnte Verwendung in Wasch-, Waschhilfs- und Reinigungsmitteln. Üblicherweise kommen die Enzyme dabei nicht als Konzentrate, sondern in Mischungen mit einem Verdünnungs- und Trägermaterial zum Einsatz. Mischt man solche Enzymzubereitungen üblichen Waschmitteln bei, so kann beim Lagern ein erheblicher Abbau der Enzymaktivität eintreten, insbesondere wenn bleichaktive Verbindungen zugegen sind. Das Aufbringen der Enzyme auf Trägersalze unter gleichzeitiger Granulation gemäß der deutschen Offenlegungsschrift DE 16 17 190 beziehungsweise durch Aufkleben mit nichtionischen Tensiden gemäß der deutschen Offenlegungsschrift DE 16 17 188 oder wäßrigen Lösungen von Celluloseethern gemäß der deutschen Offenlegungschrift DE 17 67 568 führt nicht zu einer nennenswerten Verbesserung der Lagerstabilität, da sich die empfindlichen Enzyme in solchen Aufmischungen in der Regel auf der Oberfläche der Trägersubstanz befinden. Zwar kann die Lagerstabilität der Enzyme wesentlich erhöht werden, wenn man die Enzyme mit dem Trägermaterial umhüllt beziehungsweise in dieses einbettet und anschließend durch Extrudieren, Pressen und Marumerisieren in die gewünschte Partikelform überführt, wie zum Beispiel in der deutschen Patentschrift DE 16 17 232, der deutschen Offenlegungsschrift DE 20 32 768, und den deutschen Auslegeschriften DE 21 37 042 und DE 21 37 043 beschrieben. Derartige Enzymzubereitungen besitzen jedoch nur mangelhafte Löslichkeitseigenschaften. Die ungelösten Partikel können sich im Waschgut verfangen und dieses verunreinigen bzw. sie werden ungenutzt in das Abwasser überführt. Aus der deutschen Auslegeschrift DE 1803 099 bekannte Einbettungsmittel, die aus einem Gemisch fester Säuren beziehungsweise saurer Salze und Carbonaten beziehungsweise Bicarbonaten bestehen und bei Wasserzusatz zerfallen, verbessern zwar das Lösungsvermögen, sind aber ihrerseits sehr empfindlich gegen Feuchtigkeit und erfordern daher zusätzliche Schutzmaßnahmen.

Ein weiterer Nachteil der vorgenannten Zubereitung ist darin zu sehen, daß die Enzyme nur in Form trockener Pulver verarbeitet werden können. Die üblicherweise bei der Enzymherstellung anfallenden Fermenterbrühen lassen sich in dieser Form nicht einsetzen, sondern müssen zuvor entwässert werden. An diese Voraussetzung sind auch solche Verfahren gebunden, bei denen ausschließlich leicht lösliche Trägermaterialien, wie Zucker, Stärke und Celluloseether als Bindemittel zur Herstellung von Enzymzubereitungen eingesetzt werden.

Aus der europäischen Patentschrift EP 168 526 sind Enzymgranulate bekannt, die in Wasser quellfähige Stärke, Zeolith und wasserlösliches Granulierhilfsmittel enthalten. In diesem Dokument wird ein Herstellungsverfahren für derartige Formulierungen vorgeschlagen, das im wesentlichen darin besteht, eine von unlöslichen Bestandteilen befreite Fermenterlösung aufzukonzentrieren, mit den genannten Zuschlagstoffen zu versetzen und das entstandene Gemisch zu granulieren. Das Verfahren mit dem dort vorgeschlagenen Zuschlagstoffgemisch wird vorteilhaft mit Fermentationslösungen durchgeführt, die auf einen relativ hohen Trockensubstanzgehalt, beispielsweise 55 Gew.-%, aufkonzentriert worden sind. Außerdem weisen die derart hergestellten Granulate eine so hohe Lösungs- beziehungsweise Zerfallsgeschwindigkeit unter Waschbedingungen auf, daß die Granulate teilweise schon bei der Lagerung relativ rasch zerfallen und die Enzyme desaktiviert werden.

Aus der europäischen Patentschrift EP 0 564 476 sind Enzymgranulate zum Einsatz in körnigen Wasch- und Reinigungsmitteln bekannt, die 2 Gew.-% bis 20 Gew.-% Enzym, 10 Gew.-% bis 50 Gew.-% quellfähige Stärke, 5 Gew.-% bis 50 Gew.-% wasserlösliches organisches Polymer als Granulierhilfsmittel, 10 Gew.-% bis 35 Gew.-% Getreidemehl und 3 Gew.-% bis 12 Gew.-% Wasser enthalten. Durch derartige Zuschlagstoffe wird die Enzymverarbeitung ohne größere Aktivitätsverluste möglich und auch die Lagerbeständigkeit der Enzyme in den Granulaten ist zufriedenstellend. Aus diesem Dokument ist auch bekannt, daß Natriumcarboxymethylcellulose die Zerfalls- und Dispergiergeschwindigkeit der Granulate in kalten Waschlaugen herabsetzt, während durch einen Zusatz von höhermolekularem Polyethylenglykol diese Wirkung in Richtung auf eine höhere Auflösungsgeschwindigkeit verändert werden kann. Jedoch weisen die dort beschriebenen Enzymgranulate nicht immer eine so hohe Zerfallsgeschwindigkeit auf, daß bei deren Einsatz in Waschmitteln schon in der Anfangsphase der maschinellen Wäsche genügend Enzym in der Waschflotte vorhanden ist, um enzymatisch entfernbare Anschmutzungen zu beseitigen. Als Ansatzpunkt zur weiteren Verbesserung der Löslichkeit wurde in der deutschen Patentanmeldung DE 43 10 506 der Einsatz eines speziellen Granulierhilfsmittelsystems vorgeschlagen, das Alkali-Carboxymethylcellulose mit Substitutionsgraden von 0,5 bis 1 und Polyethylenglykol und/oder Alkylbeziehungsweise Alkenylpolyethoxylat in bestimmten Mengen enthält. Bei mit Hilfe dieses Granulierhilfsmittelsystems hergestellten Enzymgranulaten ist das Problem der Einspülbarkeit in haushaltsübliche Waschmaschinen, deren Einspülkammer nicht optimal geformt und angeordnet ist, nicht vollständig zufriedenstellend gelöst.

Der vorliegenden Erfindung lag die Aufgabe zugrunde, den Ansatz zur Lösung dieser Probleme über das Granulierhilfsmittel weiterzuentwickeln und die Pulvereigenschaften, insbesondere die Löslichkeit und Einspülbarkeit der bekannten Produkte noch weiter zu verbessern, die Lagerbeständigkeit sowohl der Enzyme als auch der diese enthaltenden Granulate weiter zu erhöhen und die Aktivitätsverluste bei der Enzymverarbeitung weiter zu vermindern. Diese Aufgabe wird durch die nachfolgend geschilderte Erfindung im wesentlichen durch ein spezielles Granulierhilfsmittelsystem gelöst.

Die Erfindung betrifft ein für die Einarbeitung in insbesondere teilchenförmige Wasch- oder Reinigungsmittel geeignetes Enzymgranulat, enthaltend Enzym und anorganisches und/oder organisches Trägermaterial sowie Granulierhilfsmittel, welches dadurch gekennzeichnet ist, daß es als Granulierhilfsmittel eine phosphatierte, gegebenenfalls teilhydrolysierte Stärke enthält. Dabei wird unter phosphatierter Stärke ein Stärkederivat verstanden, bei der Hydroxylgruppen der Stärke-Anhydroglukoseeinheiten durch die Gruppe -O-P(O)(OH)₂ oder deren wasserlösliche Salze, insbesondere Alkalisalze wie Natrium- und/oder Kaliumsalze, ersetzt sind.

Unter dem mittleren Phosphatierungsgrad der Stärke ist die Zahl der veresterten, eine Phosphatgruppe tragenden Sauerstoffatome pro Saccharid-Monomer der Stärke gemittelt über alle Saccharid-Einheiten, zu verstehen. Der mittlere Phosphatierungsgrad bei vorzugsweise eingesetzten phosphatierten Stärken liegt im Bereich von 1,5 bis 2,5, da besonders bei deren Einsatz viel geringere Mengen erforderlich sind, um eine bestimmte Granulatfestigkeit zu erreichen, als bei Einsatz von Carboxymethylcellulose. Unter teilhydrolysierten Stärken sollen im Rahmen der vorliegenden Erfindung Oligo- beziehungsweise Polymere von Kohlenhydraten verstanden werden, die nach üblichen, beispielsweise säure- oder enzymkatalysierten Verfahren durch partielle Hydrolyse von Stärke zugänglich sind. Vorzugsweise handelt es sich um Hydrolyseprodukte mit mittleren Molmassen im Bereich von 440 bis 500 000. Bevorzugt sind Polysaccharide mit einem Dextrose-Equivalent (DE) im Bereich von 0,5 bis 40, insbesondere von 2 bis 30, wobei DE ein gebräuchliches Maß für die reduzierende Wirkung eines Polysaccharids im Vergleich zu Dextrose, welche ein DE von 100 besitzt, ist. Brauchbar sind nach Phosphatierung sowohl Maltodextrine (DE 3 - 20) und Trockenglukosesirupe (DE 20 - 37) als auch sogenannte Gelbdextrine und Weißdextrine mit höheren mittleren Molmassen im Bereich von etwa 2 000 bis 30 000.

Das Enzymgranulat enthält in einer bevorzugten Ausführungsform 0,01 Gew.-% bis 25 Gew.-%, insbesondere 4 Gew.-% bis 20 Gew.-%, berechnet als Trockensubstanz, Enzym, insbesondere Protease, Lipase, Amylase und/oder Cellulase, 50 Gew.-% bis 90 Gew.-% anorganisches und/oder organisches Trägermaterial und 1 Gew.-% bis 50 Gew.-% die phosphatierte Stärke enthaltendes Granulierhilfsmittelsystem sowie als Rest auf 100 Gew.-% Wasser.

Als Granulierhilfsmittelsystem enthält ein erfindungsgemäßes Enzymgranulat vorzugsweise ein Gemisch, enthaltend, jeweils bezogen auf fertiges Granulat, 0,1 Gew.-% bis 20 Gew.-%, insbesondere 0,5 Gew.-% bis 15 Gew.-% phosphatierte Stärke sowie 0,1 Gew.-% bis 15 Gew.-%, insbesondere 0,5 Gew.-% bis 10 Gew.-% Polyethylenglykol mit einer mittleren Molmasse von 200 bis 6000, 1,2-Propylenglykol und/oder eines Polyethoxylats gemäß Formel I,

R-(OCH₂CH₂)ₙ-OH I

in der R einen geradkettigen oder verzweigten Alkyl- oder Alkenylrest mit bis zu 3 C-C-Doppelbindungen mit 8 bis 22, insbesondere 12 bis 18 C-Atomen und der mittlere Ethoxylierungsgrad n eine Zahl von 10 bis 80, insbesondere von 30 bis 45 bedeutet.

Weiterhin betrifft die Erfindung ein Verfahren zur Herstellung eines derartigen Enzymgranulates mit einer Korngröße von 0,3 mm bis 3 mm durch Extrudieren eines durch Vermischen einer wäßrigen enzymhaltigen Flüssigkeit, die eine gegebenenfalls durch Mikrofiltration von unlöslichen Bestandteilen befreite, aufkonzentrierte Fermentationsbrühe sein kann, mit dem Trägermaterial und dem Granulierhilfsmittel als Zuschlagstoffen entstandenen Enzym-Vorgemischs, gegebenenfalls Sphäronisierung des Extrudats in einem Rondiergerät, gegebenenfalls Trocknung und gegebenenfalls Aufbringen eines Farbstoff oder Pigment enthaltenden Überzugs, welches dadurch gekennzeichnet ist, daß man die wäßrige enzymhaltige Flüssigkeit mit einem Zuschlagstoff vermischt, der als Granulierhilfsmittel phosphatierte Stärke oder ein Granulierhilfsmittelsystem aus phosphatierter Stärke und Polyethylenglykol und/oder Polyethoxylat gemäß Formel I enthält.

Als Enzyme kommen in erster Linie die aus Mikroorganismen, wie Bakterien oder Pilzen, gewonnenen Proteasen, Lipasen, Amylasen und/oder Cellulasen in Frage, wobei von Bacillus-Arten erzeugte Proteasen sowie ihre Gemische mit Amylasen bevorzugt sind. Sie werden in bekannter Weise durch Fermentationsprozesse aus geeigneten Mikroorganismen gewonnen, die zum Beispiel in den deutschen Offenlegungsschriften DE 19 40 488, DE 20 44 161, DE 22 01 803 und DE 21 21 397, den US-amerikanischen Patentschriften US 3 632 957 und US 4 264 738 sowie der europäischen Patentanmeldung EP 006 638 beschrieben sind. Besonders vorteilhaft kann das erfindungsgemäße Verfahren zur Konfektionierung der sehr aktiven Proteasen der sogenannten vierten Generation verwendet werden, zu denen beispielsweise Durazym® und aus den internationalen Patentanmeldungen WO 95/23221, WO 95/27049, WO 95/30010, WO 95/30011, WO 95/30743 oder WO 95/34627 bekannte Enzyme gehören, deren lagerstabile Einarbeitung in Wasch- und Reinigungsmittel oft Probleme bereitet. Erfindungsgemäß ist es möglich, die bei den Fermentationsprozessen anfallenden Brühen extrazellulärer Enzyme nach Abtrennen der unlöslichen Begleitstoffe durch Mikrofiltration sowie nachfolgende Aufkonzentration durch Ultrafiltration und gegebenenfalls anschließendes Eindampfen im Vakuum unmittelbar in lagerbeständige, weitgehend geruchlose Granulate zu überführen. Die Entstehung unerwünschter Enzymstäube und die bei zusätzlichen Trocknungsprozessen auftretenden Aktivitätsverluste werden vermieden.

Enzyme sind in den erfindungsgemäßen Granulaten vorzugsweise in Mengen von 0,01 Gew.-% bis 20 Gew.-% enthalten. Falls es sich bei dem erfindungsgemäßen Enzymgranulat um eine proteasehaltige Formulierung handelt, beträgt die Proteaseaktivität vorzugsweise 60 000 Proteaseeinheiten (PE, bestimmt nach der in Tenside 7 (1970), 125 beschriebenen Methode) bis 350 000 PE, insbesondere 140 000 PE bis 280 000 PE, pro Gramm Enzymgranulat.

Als Trägermaterialien sind im Prinzip alle organischen oder anorganischen pulverförmigen Substanzen brauchbar, welche die zu granulierenden Enzyme nicht oder nur tolerierbar wenig zerstören oder desaktivieren und unter Granulationsbedingungen stabil sind. Zu derartigen Substanzen gehören beispielsweise Stärke, Getreidemehl, Cellulosepulver, Alkalialumosilikat, insbesondere Zeolith, Schichtsilikat, zum Beispiel Bentonit oder Smectit, und wasserlösliche anorganische oder organische Salze, zum Beispiel Alkalichlorid, Alkalisulfat, Alkalicarbonat oder Alkaliacetat, wobei Natrium oder Kalium die bevorzugten Alkalimetalle sind. Bevorzugt wird ein Trägermaterialgemisch aus in Wasser quellfähiger Stärke, Cellulosepulver sowie gegebenenfalls Alkalicarbonat eingesetzt.

Bei der in Wasser quellfähigen Stärke handelt es sich vorzugsweise um Maisstärke, Reisstärke, Kartoffelstärke oder Gemische aus diesen, wobei der Einsatz von Maisstärke besonders bevorzugt ist. Quellfähige Stärke ist in den erfindungsgemäßen Enzymgranulaten vorzugsweise in Mengen von 20 Gew.-% bis 70 Gew.-%, insbesondere von 25 Gew.-% bis 60 Gew.-% enthalten. Dabei beträgt die Summe der Mengen der quellfähigen Stärke und des Mehls vorzugsweise nicht über 80 Gew.-%, insbesondere 32 Gew.-% bis 70 Gew.-%.

Bei dem im Rahmen der Erfindung geeigneten Getreidemehl, welches bekanntermaßen zur Geruchsreduktion der Enzymgranulate beitragen kann, handelt es sich insbesondere um ein aus Weizen, Roggen, Gerste oder Hafer herstellbares Produkt oder um ein Gemisch dieser Mehle, wobei Vollkornmehle bevorzugt sind. Unter einem Vollkornmehl wird hier ein nicht voll ausgemahlenes Mehl verstanden, das aus ganzen, ungeschälten Körnern hergestellt worden ist oder zumindest überwiegend aus einem derartigen Produkt besteht, wobei der Rest aus voll ausgemahlenem Mehl beziehungsweise Stärke besteht. Vorzugsweise werden handelsübliche Weizenmehl-Qualitäten, wie Type 450 oder Type 550, eingesetzt. Auch die Verwendung von Mehlprodukten der zu vorgenannten quellfähigen Stärken führenden Getreidearten ist möglich, wenn darauf geachtet wird, daß die Mehle aus den ganzen Körnern hergestellt worden sind. Derartiges Getreidemehl kann in den erfindungsgemäßen Enzymgranulaten vorzugsweise in Mengen von 10 Gew.-% bis 35 Gew.-%, insbesondere von 15 Gew.-% bis 25 Gew.-% enthalten sein. Da jedoch nun überraschenderweise gefunden wurde, daß man durch den Einsatz von phosphatierter Stärke eine tendenziell eher stärkere Geruchsreduzierung der Enzymzubereitung erreicht, als wenn man Getreidemehl einsetzt, ist es möglich, auf die Mehlkomponente des Zuschlagstoffgemisches völlig zu verzichten. Gewünschtenfalls kann durch den Einsatz von Getreidemehlproteinen wie Gluten, die man durch Extraktion der nicht eiweißhaltigen Bestandteile des Mehls gewinnen kann, die Lagerstabilität der Enzyme und die Granulierbarkeit im Rahmen des Herstellprozesses verbessert werden.

Die erfindungsgemäßen Enzymgranulate enthalten vorzugsweise 1 Gew.-% bis 50 Gew.-%, vorzugsweise 3 Gew.-% bis 25 Gew.-% des Granulierhilfsmittels beziehungsweise Granulierhilfsmittelsystems, das phosphatierte Stärke und gegebenenfalls Polyethylenglykol, 1,2-Propylenglykol und/oder Alkylpolyethoxylat enthält. In diesem Granulierhilfsmittelsystem sind vorzugsweise, jeweils bezogen auf fertiges Enzymgranulat, 1 Gew.-% bis 15 Gew.-%, insbesondere 3 Gew.-% bis 10 Gew.-% phosphatierte Stärke und bis zu 5 Gew.-%, insbesondere 0,5 Gew.-% bis 3 Gew.-% Polyethylenglykol, 1,2-Propylenglykol und/oder Polyethoxylat gemäß Formel 1 enthalten. Alkali-Carboxymethylcellulose, insbesondere höher substituierte Carboxymethylcellulose mit Substitutionsgraden bis zu 3, ist in dem erfindungsgemäßen Granulierhilfsmittelsystem vorzugsweise nicht enthalten.

Gegebenenfalls können als zusätzliche Bestandteile des Granulierhilfsmittelsystems auch weitere Cellulose- oder Stärkeether, wie Carboxymethylstärke, Methylcellulose, Hydroxyethylcellulose, Hydroxypropylcellulose sowie entsprechende Cellulosemischether, Gelatine, Casein, oben genanntes Getreidemehlprotein, Traganth, Maltodextrose, Saccharose, Invertzucker, Glukosesirup oder andere in Wasser lösliche beziehungsweise dispergierbare Oligomere oder Polymere natürlichen oder synthetischen Ursprungs verwendet werden. Brauchbare synthetische wasserlösliche Polymere sind Polyacrylate, Polymethacrylate, Copolymere der Acrylsäure mit Maleinsäure oder vinylgruppenhaltige Verbindungen, ferner Polyvinylalkohol, teilverseiftes Polyvinylacetat und Polyvinylpyrrolidon. Soweit es sich bei den vorgenannten Verbindungen um solche mit freien Carboxylgruppen handelt, liegen sie normalerweise in Form ihrer Alkalisalze, insbesondere ihrer Natriumsalze vor. Derartige zusätzliche Granulierhilfsmittel können in den erfindungsgemäßen Enzymgranulaten in Mengen bis zu 15 Gew.-%, insbesondere von 0,5 Gew.-% bis 10 Gew.-% enthalten sein.

In einer bevorzugten Ausführungsform erfindungsgemäßcr beziehungsweise nach dem erfindungsgemäßen Verfahren hergestellter Enzymgranulate enthält das Trägermaterial, jeweils bezogen auf fertiges Enzymgranulat, 10 Gew.-% bis 70 Gew.-% in Wasser quellfähige Stärke, 3 Gew.-% bis 10 Gew.-% Saccharose, bis zu 70 Gew.-%, insbesondere 10 Gew.-% bis 70 Gew.-% Getreidemehl und bis zu 10 Gew.-% Zellulosepulver.

Zur Herstellung der erfindungsgemäßen Enzymgranulate geht man vorzugsweise von Fermenterbrühen aus, die durch Mikrofiltration von unlöslichen Begleitstoffen befreit werden können. Die Mikrofiltration wird dabei vorzugsweise als Querstrom-Mikrofiltration unter Verwendung poröser Rohre mit Mikroporen größer 0,1 µm, Fließgeschwindigkeiten der Konzentratlösung von mehr als 2 m/s und einem Druckunterschied zur Permeatseite von unter 5 bar durchgeführt, wie beispielsweise in der europäischen Patentanmeldung EP 200 032 beschrieben. Anschließend wird das Mikrofiltrationspermeat vorzugsweise durch Ultrafiltration, gegebenenfalls mit anschließender Vakuumeindampfung, aufkonzentriert. Die Aufkonzentration kann dabei, wie im europäischen Patent EP 0 564 476 beschrieben, so geführt werden, daß man nur zu relativ niedrigen Gehalten an Trockensubstanz von vorzugsweise 5 Gew.-% bis 50 Gew.-%, insbesondere von 10 Gew.-% bis 40 Gew.-% gelangt. Das Konzentrat wird einem zweckmäßigerweise zuvor hergestellten trockenen, pulverförmigen bis körnigen Gemisch der oben beschriebenen Zuschlagstoffe zudosiert. Der Wassergehalt der Mischung sollte so gewählt werden, daß sie sich bei der Bearbeitung mit Rühr- und Schlagwerkzeugen in körnige, bei Raumtemperatur nicht klebende Partikel überführen und bei Anwendung höherer Drücke plastisch verformen und extrudieren läßt. Vorzugsweise werden 10 Gew.-Teile bis 50 Gew.-Teile der aufkonzentrierten Fermentationsbrühe mit 70 Gew.-Teilen bis 90 Gew.-Teilen des Trägermaterials und 0,5 Gew.-Teilen bis 10 Gew.-Teilen des Granulierhilfsmittels beziehungsweise Granulierhilfsmittelsystems vermischt.

Das rieselfähige Vorgemisch wird im Prinzip bekannter Weise anschließend in einem Kneter sowie einem angeschlossenen Extruder zu einer plastischen Masse verarbeitet, wobei als Folge der mechanischen Bearbeitung sich die Masse auf Temperaturen zwischen 40°C und 60°C, insbesondere 45°C bis 55°C erwärmen kann. Das den Extruder verlassende Gut wird durch eine Lochscheibe mit nachfolgendem Abschlagmesser geführt und dadurch zu zylinderförmigen Partikeln definierter Größe zerkleinert. Zweckmäßigerweise beträgt der Durchmesser der Bohrungen in der Lochscheibe 0,3 mm bis 3 mm, vorzugsweise 0,5 mm bis 1,5 mm. Die in dieser Form vorliegenden Partikel können anschließend getrocknet und der späteren Verwendung zugeführt werden. Es hat sich jedoch als vorteilhaft erwiesen, die den Extruder und Zerhacker verlassenden zylindrischen Partikel anschließend zu sphäronisieren, das heißt sie in geeigneten Vorrichtungen zu verrunden und zu entgraten. Ein solches Sphäronisierungsverfahren ist beispielsweise in den deutschen Auslegeschriften DE 21 37 042 und DE 21 37 043 beschrieben. Man verwendet hierzu eine Vorrichtung, die aus einem zylindrischen Behälter mit stationären, festen Seitenwänden und einer bodenseitig drehbar gelagerten Reibplatte bestehen.

Vorrichtungen dieser Art sind unter der Warenbezeichnung Marumerizer® in der Technik verbreitet.

Nach der Verrundung können die noch feuchten Kügelchen kontinuierlich oder chargenweise, vorzugsweise unter Verwendung einer Wirbelschichttrockenanlage, bei vorzugsweise 35 °C bis 60 °C und insbesondere bei einer maximalen Produkttemperatur von 45 °C, bis zu einem Restfeuchtegehalt von 4 Gew.-% bis 10 Gew.-%, vorzugsweise 5 Gew.-% bis 8 Gew.-% getrocknet werden. Nach oder vorzugsweise während der Trocknung können zusätzlich Stoffe zum Umhüllen und Beschichten der Partikel eingebracht werden. Geeignete Hüllstoffe sind insbesondere die Filmbildner unter den vorgenannten wasserlöslichen organischen Polymeren. Weiterhin lassen sich in diesem Stadium auch Farbstoffe oder Pigmente auf die Partikel aufbringen, um so eine eventuelle Eigenfarbe, die meist vom Enzymkonzentrat herrührt, zu überdecken beziehungsweise zu verändern. Als inertes und physiologisch unbedenkliches Pigment hat sich insbesondere Titandioxid bewährt, das vorzugsweise in wäßriger Dispersion eingebracht wird. Das über die Pigmentdispersion beziehungsweise über die Polymer-Lösung zugeführte Wasser wird bei der gleichzeitig vorgenommenen oder anschließend erneut erforderlichen Trocknung wieder entfernt.

Das erfindungsgemäße oder nach dem erfindungsgemäßen Verfahren erhaltene Enzymgranulat wird vorzugsweise zur Herstellung fester, insbesondere teilchenförmiger Wasch- oder Reinigungsmittel verwendet, die durch einfaches Vermischen der Enzymgranulate mit in derartigen Mitteln üblichen weiteren Pulverkomponenten erhalten werden können. Für die Einarbeitung in teilchenförmige Wasch- und Reinigungsmittel weist das Enzymgranulat vorzugsweise mittlere Korngrößen im Bereich von 0,3 mm bis 3 mm, insbesondere von 0,5 mm bis 1,5 mm auf.

Durch Sieben oder Windsichten können bei der Herstellung eventuell auftretende staubförmige Anteile sowie eventuelle Grobanteile entfernt und gegebenenfalls in den Herstellungsprozess zurückgeführt werden. Die erfindungsgemäßen Granulate enthalten vorzugsweise weniger als 5 Gew.-%, insbesondere höchstens 1 Gew.-% an Partikeln mit Korngrößen unterhalb von 0,3 mm beziehungsweise oberhalb von 1,6 mm.

Die erhaltene Enzymzubereitung besteht aus weitgehend abgerundeten, staubfreien Partikeln, die in der Regel ein Schüttgewicht von etwa 500 bis 900 Gramm pro Liter, insbesondere 650 bis 880 Gramm pro Liter, aufweisen. Ihre Enzymaktivität kann, bedingt durch den flexiblen Trockensubstanzgehalt der Brühen vor dem Vermischen mit den Zuschlagstoffen, bei Einsatz von Protease-haltigen Fermenterbrühen auf Werte im Bereich von vorzugsweise 60 000 bis 350 000 Proteaseeinheiten pro Gramm (PE/g), insbesondere 140 000 PE/g bis 280000 PE/g, eingestellt werden. Die erfindungsgemäßen Granulate zeichnen sich durch eine sehr hohe Lagerstabilität, insbesondere bei Temperaturen über Raumtemperatur und hoher Luftfeuchtigkeit, sowie eine gute Einspülbarkeit in Waschmaschinen und ein rasches Lösungsverhalten in der Waschflotte aus. Vorzugsweise setzen die erfindungsgemäßen Granulate 90 % bis 100 % ihrer Enzymaktivität innerhalb von 5 Minuten in Wasser bei 30 °C frei. Ein Beleg für die gute Einspülbarkeit ist, daß bei der Auflösung von jeweils 8 g in 2000 ml Wasser von 30 °C nach 90 Sekunden, wie unten beschrieben, ein erfindungsgemäßes oder erfindungsgemäß hergestelltes Enzymgranulat vorzugsweise einen Siebrückstand (Sieb mit Maschenweite 0,2 mm) von weniger als 3 Gew.-%, insbesondere weniger als 1,5 Gew.-%, aufweist.

### Beispiele

### Beispiel 1

Durch Fermentation von nach dem in der internationalen Patentanmeldung WO 91/02792 beschriebenen Verfahren durch Transformation einer Gensequenz aus Bacillus lentus DSM 5483 modifiziertem Bacillus licheniformis (ATCC 53926) analog dem in der deutschen Patentschrift DE 29 25 427 angegebenen Verfahren wurde eine biomassehaltige Fermenterbrühe erhalten, die ca. 65 000 Proteaseeinheiten pro Gramm (PE/g) enthielt. Diese wurde durch Dekantieren, Querstrom-Mikrofiltration, Ultrafiltation (Trenngrenze bei Molekulargewicht 10 000) und anschließendes Eindampfen im Vakuum gemäß dem im europäischen Patent EP 0 564 476 beschriebenen Vorgehen zu einem Proteasegehalt von 700 000 PE/g aufkonzentriert (**B1**). Die so aufkonzentrierte Fermenterbrühe wurde in einem mit rotierendem Schlagwerkzeug ausgerüsteten Mischer mit den in Tabelle 1 aufgeführten Zuschlägen vermischt und in einem mit einer Außenkühlung versehenen Kneter homogenisiert. Die Extrusion der plastischen Masse erfolgte mittels eines mit einer Lochscheibe (Lochdurchmesser 0,9 mm) und einem rotierenden Messer ausgerüsteten Extruder. Man erhielt die in Tabelle 1 durch ihre Zusammensetzung charakterisierten Extrudate **X1** bis **X7** gemäß der Erfindung und zum Vergleich Extrudate **V1** bis **V4**, jeweils mit Längen von 0,7 mm bis 1 mm, die in einer Sphäronisierungsvorrichtung (Marumerizer®) während einer Bearbeitungszeit von etwa 1 Minute unter gleichzeitigem Bestäuben mit pulverförmigem Calciumcarbonat (3 Gew.-%) zu abgerundeten Partikeln verformt und entgratet wurden. Das den Sphäronisator verlassende Gut wurde in einem Wirbelschichttrockner bei Temperaturen von 40 °C bis 45 °C innerhalb von 15 Minuten auf einen Wassergehalt von 6 Gew.-% getrocknet. Durch anschließendes Sieben wurden Partikel mit Teilchengrößen unter 0,4 mm und über 1,6 mm weitgehend entfernt, die dem Prozeß auf der Stufe des Vermischens mit den Zuschlagstoffen wieder zugeführt wurden. Die Enzymgranulate wurden durch Aufsprühen einer wäßrigen Titandioxidpigment-Suspension während der Wirbelschichttrocknung gecoatet. Man erhielt so die erfindungsgemäßen Enzymgranulate **P1** bis **P7** beziehungsweise zum Vergleich die Granulate **VG1** bis **VG4** mit den in Tabelle 2 angegebenen Lösezeiten und Rückstandswerten.

**Tabelle 1:**

| Enzymextrudate | | | | | | | | | | | |
|---|---|---|---|---|---|---|---|---|---|---|---|
| | **X1** | **X2** | **X3** | **X4** | **X5** | **X6** | **X7** | **V1** | **V2** | **V3** | **V4** |
| **B1** | 26 | 26 | 22 | 22 | 24 | 24 | 22 | 27 | 27 | 27 | 27 |
| Zellulosepulver ^{a)} | 3 | 3 | 3 | 3 | 3 | 3 | 3 | 3 | 3 | 3 | 3 |
| Saccharose | 6 | 6 | 6 | 6 | 6 | 6 | 6 | 6 | 6 | 6 | 6 6 |
| Weizenmehl T 450 | 16 | 16 | 16 | 16 | 16 | - | - | 14 | 16 | 16 | 16 |
| Maisstärke | 43 | 44 | 46 | 46 | 42 | 57 | 58 | 43 | 43 | 43 | 43 |
| phosphatierte Stärke^{b)} | 4 | 3 | 4 | 4 | 6 | 7 | 4 | - | - | - | - |
| Gluten^{c)} | - | - | - | - | - | - | 4 | - | - | - | - |
| CMC-I^{d)} | - | - | - | - | - | - | - | - | - | - | 3 |
| CMC-II^{e)} | - | - | - | - | - | - | - | 5 | 3 | 3 | - |
| PEG-I^{f)} | - | - | - | - | - | - | - | - | - | 2 | 2 |
| PEG-II^{g)} | 2 | 2 | 3 | - | 3 | 3 | 3 | 2 | 2 | - | - |
| Tenside^{h)} | - | - | 3 | - | - | - | - | - | - | - | - |
| | | | | | | | | | | | |

| | | | | | | | | | | | |
|---|---|---|---|---|---|---|---|---|---|---|---|
| a): Technocel® 30 (Hersteller Cellulose Füllstoff Fabrik) | | | | | | | | | | | |
| b): Pregeflo® PJ 20 (mittlerer Phosphatierungsgrad 2; Hersteller Roquette-Frere) | | | | | | | | | | | |
| c): Devital® Weizenkleber (Hersteller Roquette-Frere) | | | | | | | | | | | |
| d): Carbocel® 300 (Substitutionsgrad 0,65-0,75; Hersteller Lamberti CMC) | | | | | | | | | | | |
| e): Carbocel® 500 (Substitutionsgrad 0,85-0,95; Hersteller Lamberti CMC) | | | | | | | | | | | |
| f): Polyethylenglykol, mittleres Molekulargewicht 400 | | | | | | | | | | | |
| g): Polyethylenglykol, mittleres Molekulargewicht 2000 | | | | | | | | | | | |
| h): 40-fach ethoxylierter Talgfettalkohol (Hersteller Henkel) | | | | | | | | | | | |

### Beispiel 2

Zur Bestimmung des Rückstandswertes wurden 1000 ml Wasser (16 °dH. bei 30 °C temperiert) in ein 2000 ml-Becherglas (hohe Form) gegeben, ein Laborrührer mit Propellerrührkopf wurde 1,5 cm vom Becherglasboden entfernt zentriert fixiert und mit 800 Umdrehungen pro Minute in Bewegung gesetzt. 8 g des zu testenden Granulats wurden eingestreut und 90 Sekunden gerührt. Anschließende wurde die im Becherglas befindliche Flüssigkeit durch ein Sieb (Maschenweite 0,2 mm) mit bekanntem Gewicht gegossen, das Becherglas mit möglichst wenig kaltem Wasser nachgespült und das Sieb nach Trocknen bei 40 °C bis zur Gewichtskonstanz ausgewogen. Es ergaben sich die in Tabelle 2 angegebenen Siebrückstände (Doppelbestimmung).

**Tabelle 2:**

| Siebrückstände der Enzymgranulate | |
|---|---|
| Enzymgranulat | Siebrückstand [Gew.-%] |
| **P1** | 0,6 |
| **P2** | 0,1 |
| **P3** | 0,1 |
| **P4** | 0,2 |
| **P5** | 0.1 |
| **P6** | 0.8 |
| **P7** | 1,2 |
| **VG1** | 41 |
| **VG2** | 23 |
| **VG3** | 36 |
| **VG4** | 17 |

### Beispiel 3

Die erfindungsgemäßen und die zum Vergleich hergestellten Enzymgranulate aus Beispiel 1 wurden, wie in der europäischen Patentschrift EP 0 564 476 beschrieben, unter standardisierten Bedingungen aufgelöst. Es ergaben sich die in Tabelle 3 angegebenen Enzymaktivitäten in der Lösung nach 5 Minuten Rührzeit bei 30 °C in % des Endwertes (= keine zeitliche Änderung mehr zu beobachten).

**Tabelle 3:**

| Enzymaktivität nach 5 Minuten | |
|---|---|
| Enzymgranulat | Aktivität [%] |
| **P1** | 96 |
| **P2** | 95 |
| **P3** | 100 |
| **P4** | 100 |
| **P5** | 100 |
| **P6** | 98 |
| **P7** | 100 |
| **VG1** | 82 |
| **VG2** | 88 |
| **VG3** | 85 |
| **VG4** | 93 |

## Patentansprüche

1. Für die Einarbeitung in Wasch- oder Reinigungsmittel geeignetes Enzymgranulat, enthaltend Enzym und anorganisches und/oder organisches Trägermaterial sowie Granulierhilfsmittel, dadurch gekennzeichnet, daß es als Granulierhilfsmittel eine phosphatierte Stärke enthält.

2. Enzymgranulat nach Anspruch 1, dadurch gekennzeichnet, daß der mittlere Phosphatierungsgrad der phosphatierten Stärke im Bereich von 1,5 bis 2,5 liegt.

3. Enzymgranulat nach Anspruch 1 oder 2, dadurch gekennzeichnet, daß es 0,01 Gew.-% bis 20 Gew.-%, berechnet als Trockensubstanz, Enzym, insbesondere Protease, Lipase, Amylase und/oder Cellulase, 50 Gew.-% bis 90 Gew.-% anorganisches und/oder organisches Trägermaterial und 1 Gew.-% bis 50 Gew.-% die phosphatierte Stärke enthaltendes Granulierhilfsmittelsystem sowie als Rest auf 100 Gew.-% Wasser enthält.

4. Enzymgranulat nach einem der Ansprüche 1 bis 3, dadurch gekennzeichnet, daß es als Granulierhilfsmittelsystem ein Gemisch, enthaltend, jeweils bezogen auf fertiges Granulat, 0,1 Gew.-% bis 20 Gew.-%, insbesondere 0,5 Gew.-% bis 15 Gew.-% phosphatierte Stärke und 0,1 Gew.-% bis 15 Gew.-%, insbesondere 0,5 Gew.-% bis 10 Gew.-% Polyethylenglykol mit einer mittleren Molmasse von 200 bis 6000, 1,2-Propylenglykol und/oder eines Polyethoxylats gemäß Formel I,
R-(OCH₂CH₂)ₙ-OH I
in der R einen geradkettigen oder verzweigten Alkyl- oder Alkenylrest mit bis zu 3 C-C-Doppelbindungen mit 8 bis 22 C-Atomen und der mittlere Ethoxylierungsgrad n eine Zahl von 10 bis 80 bedeutet, enthält.

5. Enzymgranulat nach einem der Ansprüche 1 bis 4, dadurch gekennzeichnet, daß das Trägermaterial, bezogen auf Enzymgranulat, 20 Gew.-% bis 70 Gew.-%, insbesondere 25 Gew.-% bis 60 Gew.-% in Wasser quellfähige Stärke, insbesondere Maisstärke, enthält.

6. Enzymgranulat nach einem der Ansprüche 1 bis 5, dadurch gekennzeichnet, daß das Trägermaterial, bezogen auf Enzymgranulat, 10 Gew.-% bis 35 Gew.-%, insbesondere 15 Gew.-% bis 25 Gew.-% Getreidemehl, insbesondere Weizenmehl, Roggenmehl, Gerstenmehl, Hafermehl und deren Gemische, bis zu 10 Gew.-%, insbesondere 2 Gew.-% bis 6 Gew.-% Cellulose, Schichtsilikat, Alkalicarbonat, Alkalichlorid, Alkalisulfat und/oder Alkaliacetat enthält und/oder das Granulierhilfsmittel zusätzlich bis zu 15 Gew.-%, insbesondere 0,5 Gew.-% bis 10 Gew.-% wasserlösliche beziehungsweise -dispergierbare Oligomere oder Polymere natürlichen oder synthetischen Ursprungs enthält.

7. Enzymgranulat nach einem der Ansprüche 1 bis 5, dadurch gekennzeichnet, daß das Trägermaterial frei von Getreidemehl ist.

8. Enzymgranulat nach einem der Ansprüche 1 bis 7, dadurch gekennzeichnet, daß es Protease mit einer Aktivität von 60 000 PE bis 350 000 PE, insbesondere 140 000 PE bis 280 000 PE, pro Gramm Enzymgranulat enthält.

9. Enzymgranulat nach einem der Ansprüche 1 bis 8, dadurch gekennzeichnet, daß es eine mittlere Korngröße von 0,3 mm bis 3 mm, insbesondere von 0,5 mm bis 1,5 mm aufweist und weniger als 5 Gew.-%, insbesondere höchstens 1 Gew.-% an Partikeln mit Korngrößen unterhalb von 0,3 mm und weniger als 5 Gew.-%, insbesondere höchstens 1 Gew.-% an Partikeln mit Korngrößen oberhalb von 1,6 mm enthält.

10. Enzymgranulat nach einem der Ansprüche 1 bis 9, dadurch gekennzeichnet, daß es in Wasser bei 30 °C mindestens 90 % seiner Enzymaktivität innerhalb von 5 Minuten freisetzt.

11. Enzymgranulat nach einem der Ansprüche 1 bis 10, dadurch gekennzeichnet, daß es bei der Auflösung von jeweils 8 g in 2000 ml Wasser von 30 °C nach 90 Sekunden einen Siebrückstand (Sieb mit Maschenweite 0,2 mm) von weniger als 3 Gew.-%, insbesondere weniger als 1,5 Gew.-%, aufweist.

12. Verfahren zur Herstellung eines für die Einarbeitung in Wasch- oder Reinigungsmittel geeigneten Enzymgranulates mit einer Korngröße von 0,3 mm bis 3 mm durch Extrudieren eines durch Vermischen einer wäßrigen enzymhaltigen Flüssigkeit mit anorganischem und/oder organischem Trägermaterial und Granulierhilfsmittel als Zuschlagstoffen entstandenen Enzym-Vorgemischs, gegebenenfalls Sphäronisierung des Extrudats in einem Rondiergerät, gegebenenfalls Trocknung und gegebenenfalls Aufbringen eines Farbstoff oder Pigment enthaltenden Überzugs, dadurch gekennzeichnet, daß man die wäßrige enzymhaltige Flüssigkeit mit einem trägermaterialhaltigen Zuschlagstoff vermischt, der als Granulierhilfsmittel phosphatierte Stärke oder ein Granulierhilfsmittelsystem aus phosphatierter Stärke und Polyethylenglykol und/oder Polyethoxylat gemäß Formel I enthält.

13. Verfahren nach Anspruch 12, dadurch gekennzeichnet, daß die wäßrige enzymhaltige Flüssigkeit eine gegebenenfalls durch Mikrofiltration von unlöslichen Bestandteilen befreite, aufkonzentrierte Fermenterbrühe ist.

14. Verfahren nach Anspruch 12 oder 13, dadurch gekennzeichnet, daß man 10 Gew.-Teile bis 50 Gew.-Teile der aufkonzentrierten Fermentationsbrühe mit 70 Gew.-Teile bis 90 Gew.-Teile des Trägermaterials und 0,5 Gew.-Teile bis 10 Gew.-Teile des Granulierhilfsmittels beziehungsweise Granulierhilfsmittelsystems vermischt.

15. Verfahren nach einem der Ansprüche 12 bis 14, dadurch gekennzeichnet, daß das Trägermaterial, jeweils bezogen auf fertiges Enzymgranulat, 10 Gew.-% bis 70 Gew.-% in Wasser quellfähige Stärke, 3 Gew.-% bis 10 Gew.-% Saccharose, bis zu 70 Gew.-%, insbesondere 10 Gew.-% bis 70 Gew.-% Getreidemehl und bis zu 10 Gew.-% Zellulosepulver enthält.

16. Verfahren nach einem der Ansprüche 12 bis 15, dadurch gekennzeichnet, daß man die Extrusion mit Hilfe einer Lochplatte, deren Löcher einen Durchmesser von 0,7 mm bis 1,2 mm aufweisen, durchführt.

17. Verfahren nach einem der Ansprüche 12 bis 16, dadurch gekennzeichnet, daß man das Extrudat verrundet nach der Verrundung das Granulat bei Temperaturen von 35 °C bis 50 °C auf einen Wassergehalt von 4 Gew.-% bis 10 Gew.-% trocknet.

18. Verwendung eines Enzymgranulats gemäß einem der Ansprüche 1 bis 11 oder erhalten nach dem Verfahren gemäß einem der Ansprüche 12 bis 17 zur Herstellung fester, insbesondere teilchenförmiger Wasch- oder Reinigungsmittel.

## Claims

1. Enzyme granules suitable for incorporation in detergents or cleaners containing enzyme and inorganic and/or organic carrier material and granulation auxiliary, characterized in that they contain a phosphated starch as the granulation auxiliary.

2. Enzyme granules as claimed in claim 1, characterized in that the mean degree of phosphation of the phosphated starch is in the range from 1.5 to 2.5.

3. Enzyme granules as claimed in claim 1 or 2, characterized in that they contain 0.01% by weight to 20% by weight, expressed as dry matter, of enzyme, more particularly protease, lipase, amylase and/or cellulase, 50% by weight to 90% by weight of inorganic and/or organic carrier material and 1% by weight to 50% by weight of granulation auxiliary system containing the phosphated starch, the balance to 100% by weight being water.

4. Enzyme granules as claimed in any of claims 1 to 3, characterized in that they contain a mixture containing - based on the final granules - 0.1% by weight to 20% by weight and, more particularly, 0.5% by weight to 15% by weight of phosphated starch and 0.1% by weight to 15% by weight and, more particularly, 0.5% by weight to 10% by weight of polyethylene glycol with an average molecular weight of 200 to 6,000, 1,2-propylene glycol and/or a polyethoxylate corresponding to formula I
R-(OCH₂CH₂)ₙ-OH I
where R is a linear or branched alkyl or alkenyl group containing up to 3 C-C double bonds and 8 to 22 carbon atoms and the average degree of ethoxylation n is a number of 10 to 80,
as the granulation auxiliary system.

5. Enzyme granules as claimed in any of claims 1 to 4, characterized in that, based on the enzyme granules, the carrier material contains 20% by weight to 70% by weight and, more particularly, 25% by weight to 60% by weight of starch swellable in water, more particularly corn starch.

6. Enzyme granules as claimed in any of claims 1 to 5, characterized in that based on enzyme granules, the carrier material contains 10% by weight to 35% by weight and, more particularly, 15% by weight to 25% by weight of cereal flour, more particularly wheat flour, rye flour, barley flour, oat flour and mixtures thereof, up to 10% by weight and, more particularly, 2% by weight to 6% by weight of cellulose, layer silicate, alkali metal carbonate, alkali metal chloride, alkali metal sulfate and/or alkali metal acetate and/or the granulation auxiliary additionally contains up to 15% by weight and, more particularly, 0.5% by weight to 10% by weight of water-soluble or water-dispersible oligomers or polymers of natural or synthetic origin.

7. Enzyme granules as claimed in any of claims 1 to 5, characterized in that the carrier material is free from cereal flour.

8. Enzyme granules as claimed in any of claims 1 to 7, characterized in that they contain protease with an activity of 60,000 PU to 350,000 PU and, more particularly, 140,000 PU to 280,000 PU per gram of enzyme granules.

9. Enzyme granules as claimed in any of claims 1 to 8, characterized in that they have a mean particle size of 0.3 mm to 3 mm and, more particularly, 0.5 mm to 1.5 mm and contain less than 5% by weight and, more particularly, at most 1 % by weight of particles with particle sizes below 0.3 mm and less than 5% by weight and, more particularly, at most 1 % by weight of particles with particle sizes above 1.6 mm.

10. Enzyme granules as claimed in any of claims 1 to 9, characterized in that they release at least 90% of their enzyme activity in 5 minutes in water at 30°C.

11. Enzyme granules as claimed in any of claims 1 to 10, characterized in that, when dissolved in quantities of 8 g in 2,000 ml of water at 30°C, their sieve residue after 90 seconds (0.2 mm mesh sieve) is less than 3% by weight and, more particularly, less than 1.5% by weight.

12. A process for the production of enzyme granules with a particle size of 0.3 mm to 3 mm by extruding an enzyme "compound" prepared by mixing an aqueous enzyme-containing liquid with inorganic and/or organic carrier material and granulation auxiliary as additives, optionally spheronizing the extrudate in a spheronizer, optionally drying and optionally applying a dye- or pigment-containing coating, characterized in that the aqueous enzyme-containing liquid is mixed with a carrier-containing additive which contains phosphated starch or a granulation auxiliary system of phosphated starch and polyethylene glycol and/or polyethoxylate corresponding to formula I as the granulation auxiliary.

13. A process as claimed in claim 12, characterized in that the aqueous enzyme-containing liquid is a concentrated fermenter broth optionally freed from insoluble constituents by microfiltration.

14. A process as claimed in claim 12 or 13, characterized in that 10 parts by weight to 50 parts by weight of the concentrated fermentation broth are mixed with 70 parts by weight to 90 parts by weight of the carrier material and 0.5 part by weight to 10 parts by weight of the granulation auxiliary or granulation auxiliary system.

15. A process as claimed in any of claims 12 to 14, characterized in that, based on the final enzyme granules, the carrier material contains 10% by weight to 70% by weight of starch swellable in water, 3% by weight to 10% by weight of sucrose, up to 70% by weight and, more particularly, 10% by weight to 70% by weight of cereal flour and up to 10% by weight of cellulose powder.

16. A process as claimed in any of claims 12 to 15, characterized in that the extrusion step is carried out using a multiple-bore extrusion die with a bore diameter of 0.7 mm to 1.2 mm.

17. A process as claimed in any of claims 12 to 16, characterized in that the extrudate is spheronized and, after spheronizing, the granules are dried at temperatures of 35°C to 50°C to a water content of 4% by weight to 10% by weight.

18. The use of the enzyme granules claimed in any of claims 1 to 11 or obtained by the process claimed in any of claims 12 to 17 for the production of solid detergents or cleaners, more particularly particulate detergents or cleaners.

## Revendications

1. Granulé enzymatique approprié à l'incorporation dans des produits de lavage ou de nettoyage renfermant une enzyme, une matière porteuse inorganique et/ou organique et un adjuvant de granulation, caractérisé en ce qu'il renferme comme adjuvant de granulation, un amidon phosphaté.

2. Granulé enzymatique selon la revendication 1, caractérisé en ce que le degré de phosphatation moyen de l'amidon phosphaté est compris dans l'intervalle de 1,5 à 2,5.

3. Granulé enzymatique selon la revendication 1 ou 2, caractérisé en ce qu'il renferme 0,01 à 20 % en poids, calculés comme substance sèche, d'enzyme, en particulier de protéase, de lipase, d'amylase et/ou de cellulase, 50 à 90 % en poids de matière porteuse inorganique et/ou organique et 1 à 50 % en poids de système d'adjuvant de granulation contenant l'amidon phosphaté, ainsi que de l'eau comme reste pour faire 100 %.

4. Granulé enzymatique selon une des revendications 1 à 3, caractérisé en ce qu'il renferme comme système d'adjuvant de granulation, un mélange contenant (dans chaque cas par rapport au granulé prêt à l'emploi) 0,1 à 20 % en poids, en particulier 0,5 à 15 % en poids d'amidon phosphaté ainsi que 0,1 à 15 % en poids, en particulier 0,5 à 10 % en poids de polyéthylèneglycol possédant une masse molaire moyenne comprise entre 200 et 6000, de 1,2-propylèneglycol et/ou d'un polyéthoxylate selon la formule I,
R-(OCH₂CH₂)ₙ-OH (I)
dans laquelle R représente un radical alkyle ou alcényle à chaîne droite ou ramifiée, comportant jusqu'à 3 doubles liaisons C-C avec 8 à 22 atomes de C, et le degré d'éthoxylation moyen n correspond à un nombre de 10 à 80.

5. Granulé enzymatique selon une des revendications 1 à 4, caractérisé en ce que la matière porteuse renferme, par rapport au granulé enzymatique, 20 à 70 % en poids, en particulier 25 à 60 % en poids d'amidon gonflable dans l'eau en particulier d'amidon de maïs.

6. Granulé enzymatique selon une des revendications 1 à 5, caractérisé en ce que la matière porteuse renferme, par rapport au granulé enzymatique, 10 à 35 % en poids, en particulier de 15 à 25 % en poids de farine de céréales, en particulier de farine de froment, de seigle, d'orge ou d'avoine et de mélanges de celles-ci, jusqu'à 10 % en poids, en particulier 2 à 6 % en poids de cellulose, de silicate lamellaire, de carbonate, de chlorure, de sulfate et/ou d'acétate de métal alcalin, et/ou en ce que l'adjuvant de granulation contient en plus jusqu'à 15 % en poids, en particulier 0,5 à 10 % en poids d'oligomères ou de polymères d'origine naturelle ou synthétique, solubles ou dispersibles dans l'eau.

7. Granulé enzymatique selon une des revendications 1 à 5, caractérisé en ce que la matière porteuse est exempte de farine de céréales.

8. Granulé enzymatique selon une des revendications 1 à 7, caractérisé en ce qu'il renferme une protéase possédant une activité de 60 000 à 350 000 UP, en particulier de 140 000 à 280 000 UP, par gramme de granulé enzymatique.

9. Granulé enzymatique selon une des revendications 1 à 8, caractérisé en ce qu'il présente une grosseur de grain moyenne de 0,3 à 3 mm, en particulier de 0,5 à 1,5 mm, et moins de 5 % en poids, en particulier au maximum 1 % en poids de particules avec une grosseur de grain inférieure à 0,3 mm, et moins de 5 % en poids, en particulier au maximum 1 % en poids de particules avec une grosseur de grain supérieure à 1,6 mm.

10. Granulé enzymatique selon une des revendications 1 à 9, caractérisé en ce qu'il libère au moins 90 % de son activité enzymatique dans les 5 minutes dans de l'eau à 30 °C.

11. Granulé enzymatique selon une des revendications 1 à 10, caractérisé en ce qu'il présente en cas de dissolution de chaque fois 8 g dans 2000 ml d'eau à 30 °C, après 90 secondes un refus de tamis (tamis avec une largeur de maille de 0,2 mm) inférieur à 3 % en poids, en particulier inférieur à 1,5 % en poids.

12. Procédé de production d'un granulé enzymatique approprié à l'incorporation dans des produits de lavage ou de nettoyage avec une grosseur de grain de 0,3 à 3 mm, par extrusion d'un prémélange d'enzymes obtenu par mélangeage d'un liquide aqueux renfermant les enzymes avec la matière porteuse et l'adjuvant de granulation comme agrégats, éventuellement sphéronisation du produit d'extrusion dans un appareil à arrondir, éventuellement séchage et éventuellement application d'un revêtement renfermant un colorant ou un pigment, qui est caractérisé en ce que l'on mélange le liquide aqueux renfermant les enzymes avec un agrégat contenant la matière porteuse, qui renferme comme adjuvant de granulation, de l'amidon phosphaté ou un système d'adjuvant de granulation constitué d'amidon phosphaté et de polyéthylèneglycol et/ou de polyéthoxylate selon la formule 1.

13. Procédé selon la revendication 12, caractérisé en ce que le liquide aqueux renfermant les enzymes est un bouillon de fermentateur concentré, éventuellement débarrassé par microfiltration des constituants insolubles

14. Procédé selon la revendication 12 ou 13, caractérisé en ce que l'on mélange 10 à 50 parties en poids du bouillon de fermentation concentré avec 70 à 90 parties en poids de la matière porteuse et 0,5 à 10 parties en poids de l'adjuvant de granulation ou du système d'adjuvant de granulation.

15. Procédé selon une des revendications 12 à 14, caractérisé en ce que la matière porteuse renferme, dans chaque cas par rapport au granulé enzymatique prêt à l'emploi, 10 à 70 % en poids d'amidon gonflable dans l'eau, 3 à 10 % en poids de saccharose, jusqu'à 70 % en poids, en particulier entre 10 et 70 % en poids de farine de céréales et jusqu'à 10 % en poids de poudre de cellulose.

16. Procédé selon une des revendications 12 à 15, caractérisé en ce que l'on opère l'extrusion à l'aide d'un disque perforé dont les trous présentent un diamètre de 0,7 à 1,2 mm.

17. Procédé selon une des revendications 12 à 16, caractérisé en ce que l'on arrondit le produit d'extrusion, que l'on sèche après l'arrondissage à des températures de 35 à 50 °C jusqu'à une teneur en eau de 4 à 10 % en poids.

18. Utilisation d'un granulé enzymatique conforme à une des revendications 1 à 11 ou obtenu selon le procédé conforme à une des revendications 12 à 17 pour la production de produits de lavage ou de nettoyage, en particulier sous forme de particules.
